# EUROPÄISCHE PATENTSCHRIFT

(11) **EP 3 590 871 B1**
(45) Veröffentlichungstag und Bekanntmachung des Hinweises auf die Patenterteilung: **02.11.2022**
(21) Anmeldenummer: 19180483.0
(22) Anmeldetag: 17.06.2019
(51) Int. Cl.: B65G 43/08, B65G 47/76, B30B 11/00, B30B 11/08, B30B 15/32

(54) **RUNDLÄUFERTABLETTENPRESSE SOWIE VERFAHREN ZUM BETÄTIGEN EINER WEICHE EINER RUNDLÄUFERTABLETTENPRESSE**
ROTARY TABLET PRESS AND METHOD FOR OPERATING A SWITCH OF A ROTARY TABLET PRESS
PRESSE À COMPRIMÉS ROTATIVE ET PROCÉDÉ D'ACTIONNEMENT D'UN DISPOSITIF D'AIGUILLAGE D'UNE PRESSE À COMPRIMÉS ROTATIVE

(30) Priorität: 04.07.2018 DE 102018116143
(43) Veröffentlichungstag der Anmeldung: 08.01.2020
(73) Patentinhaber: Fette Compacting GmbH, 21493 Schwarzenbek (DE)
(72) Erfinder: Meißner, Friedrich, 21493 Schwarzenbek (DE); Lüdemann, Stefan, 21035 Hamburg (DE)
(74) Vertreter: Hauck Patentanwaltspartnerschaft mbB

(56) Entgegenhaltungen:
- EP-A2- 2 368 704
- WO-A1-2008/038070
- CN-U- 205 112 500
- US-A- 4 643 291

## Beschreibung

Die Erfindung betrifft eine Rundläufertablettenpresse, umfassend einen mittels eines Drehantriebs drehbaren Rotor, wobei der Rotor eine obere Stempelführung für obere Stempel der Rundläufertablettenpresse und eine untere Stempelführung für untere Stempel der Rundläufertablettenpresse sowie eine zwischen den Stempelführungen angeordnete Matrizenschreibe aufweist, wobei die Stempel mit Kavitäten der Matrizenscheibe zusammenwirken, weiter umfassend eine Fülleinrichtung, durch die zu verpressendes Füllmaterial in die Kavitäten der Matrizenscheibe gefüllt wird, weiter umfassend mindestens eine obere Druckeinrichtung und mindestens eine untere Druckeinrichtung, die im Betrieb mit den oberen Stempeln und mit den unteren Stempeln zum Verpressen des Füllmaterials in den Kavitäten der Matrizenscheibe zu Tabletten zusammenwirken, weiter umfassend eine Auswurfeinrichtung, in der in den Kavitäten erzeugte Tabletten ausgeworfen werden, und umfassend einen Tablettenablauf, dem die ausgeworfenen Tabletten zugeführt werden, wobei in dem Tablettenablauf mindestens eine Weiche angeordnet ist, wobei eine Steuereinrichtung vorgesehen ist, und wobei eine Antriebseinrichtung vorgesehen ist, die die Weiche zwischen einer ersten Schaltstellung, in der Tabletten einem ersten Tablettenausgang zugeleitet werden und mindestens einer zweiten Schaltstellung, in der Tabletten mindestens einem zweiten Tablettenausgang zugeleitet werden, bewegt.

Die Erfindung betrifft außerdem ein Verfahren zum Betätigen einer Weiche eines Tablettenablaufs einer Rundläufertablettenpresse, bei dem die Weiche zwischen einer ersten Schaltstellung, in der Tabletten einem ersten Tablettenausgang zugeleitet werden, und mindestens einer zweiten Schaltstellung, in der Tabletten mindestens einem zweiten Tablettenausgang zugeleitet werden, bewegt wird.

Rundläufertablettenpressen weisen eine Vielzahl von oberen und unteren Stempeln auf, die jeweils paarweise einer Kavität einer Matrizenscheibe eines Rotors der Rundläufertablettenpresse zugeordnet sind. Im Zuge der Drehung des Rotors werden die Kavitäten mit dem zu verpressenden Füllmaterial befüllt. In mindestens einer Druckeinrichtung werden die oberen und unteren Stempel in den Kavitäten zum Verpressen des Füllmaterials zu Tabletten gegeneinander gedrückt. Nach dem Verpressen werden die Tabletten in der Regel durch die Unterstempel aus den Kavitäten ausgestoßen und beispielsweise durch einen Abstreifer einem Tablettenablauf zugeführt. Die abgeführten Tabletten werden beispielsweise anhand von Messwerten einer Sensorik der Rundläufertablettenpresse unterschiedlichen Tablettenausgängen zugeleitet, beispielsweise Tablettenausgängen für Guttabletten, für Schlechttabletten oder für Tabletten eines Musterzugs. Zum Leiten der Tabletten in unterschiedliche Tablettenausgänge befinden sich in Tablettenabläufen von Rundläufertablettenpressen regelmäßig Weichen. Solche Weichen können zum Beispiel eine schwenkbar zwischen beispielsweise zwei Schaltstellungen bewegbare Weichenfahne aufweisen, die die Tabletten je nach Schaltstellung zu einem ersten Tablettenausgang oder einem zweiten Tablettenausgang leitet.

Es ist bekannt, das Erreichen der jeweiligen Endlagen der Weiche mittels Sensoren zu detektieren. Für eine Weiche mit zwei Schaltstellungen sind in der Regel zwei Sensoren für das Detektieren der jeweiligen Endlage vorgesehen. Sofern ein Nichterreichen einer Endlage durch die Sensoren erkannt wird, wird eine entsprechende Fehlermeldung ausgegeben.

Entsprechend der hohen Produktionskapazitäten moderner Rundläufertablettenpressen fließt durch den Tablettenablauf ein erheblicher Strom von Tabletten. Dabei fließen die Tabletten mit nur geringem Abstand zwischen einander. Dies kann beim Schalten der Weiche in der Praxis zu einem Einklemmen von Tabletten zwischen der Weiche, beispielsweise dem freien Ende einer Weichenfahne, und einer gegenüberliegenden Wand des Tablettenablaufs führen. Die Weiche kann entsprechend ihre Zielschaltstellung nicht erreichen. Dies wiederum kann zu einem Tablettenstau in dem Tablettenablauf und zu einem Abschalten der Rundläufertablettenpresse aufgrund einer Weichenfehlfunktion führen. Dies schränkt die Verfügbarkeit der Rundläufertablettenpresse erheblich ein und der manuelle Aufwand für eine Bedienperson zum Beheben der Fehlfunktion ist hoch. So ist es für das Beheben eines Tablettenstaus erforderlich, das Gehäuse der Rundläufertablettenpresse zu öffnen, was insbesondere bei toxischen Pressmaterialien zu einem erhöhten Gesundheitsrisiko führt. Weiter ist regelmäßig eine Demontage von Komponenten, wie der Weichenantriebe, der Säule Luft, Abdeckungen etc. erforderlich, um den Bereich des Tablettenstaus zugänglich zu machen. Die gestauten Tabletten müssen entfernt werden, ebenso etwaige Bruchstücke und der Tablettenablauf muss gereinigt werden. Weiterhin muss geprüft werden, ob versehentlich Schlechttabletten oder Bruchstücke in die Gutproduktion gelangt sind. Anschließend müssen die demontierten Komponenten wieder montiert werden und die Rundläufertablettenpresse muss wieder in Betrieb genommen werden.

Aus WO 2008/038070 A1 ist eine Rundläufertablettenpresse mit einer durch eine Antriebseinrichtung und eine Steuereinrichtung zwischen einer Ausgangsschaltstellung und einer Zielschaltstellung bewegbaren Weiche bekannt. Die WO 2008/038070 A1 offenbart eine Rundläufertablettenpresse gemäß dem Oberbegriff des Anspruchs 1.

Weiterhin ist aus EP 2 368 704 A3 ein Tablettenablauf zum Abführen von in einer Tablettenpresse verpressten Tabletten bekannt. Der Tablettenablauf weist eine Weiche auf. Die Weiche umfasst einen Führungskanal für Tabletten, wobei ein Führungsgetriebe vorgesehen ist, mit dem der Führungskanal zwischen einer ersten, einen Hauptablaufkanal mit einem ersten Ablaufkanal verbindenden Stellung und einer zweiten, den Hauptablaufkanal mit einem zweiten Ablaufkanal verbindenden Stellung bewegbar ist.

Ausgehend von dem erläuterten Stand der Technik liegt der Erfindung die Aufgabe zugrunde, eine Rundläufertablettenpresse und ein Verfahren der eingangs genannten Art bereitzustellen, mit denen auch bei hohen Produktionskapazitäten die Verfügbarkeit der Rundläufertablettenpresse maximiert und der Aufwand und die Gesundheitsgefährdung für Bedienpersonen minimiert werden.

Die Erfindung löst die Aufgabe durch die unabhängigen Ansprüche 1 und 13. Vorteilhafte Ausgestaltungen finden sich in den abhängigen Ansprüchen, der Beschreibung und den Figuren.

Für eine Rundläufertablettenpresse der eingangs genannten Art löst die Erfindung die Aufgabe dadurch, dass die Steuereinrichtung dazu ausgebildet ist, die Antriebseinrichtung zum Bewegen der Weiche aus einer Ausgangsschaltstellung in eine Zielschaltstellung derart anzusteuern, dass die Weiche ausgehend von der Ausgangsschaltstellung mit einer Bewegungsgeschwindigkeit in Richtung der Zielschaltstellung bewegt wird, wobei die Bewegungsgeschwindigkeit der Weiche vor Erreichen der Zielschaltstellung verringert wird.

Für ein Verfahren der eingangs genannten Art löst die Erfindung die Aufgabe dadurch, dass die Weiche zum Bewegen aus einer Ausgangsschaltstellung in eine Zielschaltstellung ausgehend von der Ausgangsstellung mit einer Bewegungsgeschwindigkeit in Richtung der Zielschaltstellung bewegt wird, wobei die Bewegungsgeschwindigkeit der Weiche vor Erreichen der Zielschaltstellung verringert wird.

Die Weiche kann zwischen einer ersten Schaltstellung, die beispielsweise eine Ausgangsschaltstellung bilden kann, und mindestens einer zweiten Schaltstellung, die beispielsweise eine Zielschaltstellung bilden kann, bewegt werden. Die erste Schaltstellung und die mindestens eine zweite Schaltstellung bzw. die Ausgangsschaltstellung und die Zielschaltstellung sind jeweils Endstellungen der Weiche bzw. einer Weichenfahne der Weiche. In diesen Endstellungen ist eine Trennung zwischen Ablaufkanälen der Weiche und damit eine Führung des Tablettenstroms in den gewünschten Tablettenausgang sichergestellt. In mindestens einer Endstellung kann die Weiche bzw. eine Weichenfahne beispielsweise an einer Innenwand eines Ablaufkanals des Tablettenablaufs anliegen. Sie kann aber auch in mindestens einer Endstellung einen Teil einer Trennwand zwischen unterschiedlichen Ablaufkanälen des Tablettenablaufs bilden.

Bei der erfindungsgemäßen Weiche ist eine Steuereinrichtung vorgesehen, die ein Schaltsignal zum Bewegen der Weiche zwischen einer ersten Schaltstellung und mindestens einer zweiten Schaltstellung ausgibt. Die Steuereinrichtung kann das Schaltsignal zum Beispiel aufgrund von Messwerten einer Sensorik der Tablettenpresse oder aufgrund von Eingaben zum Ziehen eines Musterzugs abgeben. Beispielsweise können Sensoren der Rundläufertablettenpresse unerlaubte Parameterabweichungen von hergestellten Tabletten erkennen. Diese Messwerte können an der Steuereinrichtung anliegen und die Steuereinrichtung kann entsprechend ein Schaltsignal zum Bewegen der Weiche in eine Schaltstellung ausgeben, in der die entsprechenden Tabletten einem Tablettenausgang für Schlechttabletten zugeführt werden. Auch kann die Steuereinrichtung bei Vorliegen eines Signals für das Ziehen eines Musterzugs ein Schaltsignal zum Bewegen der Weiche in eine Schaltstellung ausgeben, in der eine vorgegebene Tablettenmenge einem Tablettenausgang für einen Musterzug, also eine Stichprobenuntersuchung, zugeführt wird. Die Steuereinrichtung kann gleichzeitig die Steuereinrichtung zum Steuern des Betriebs der Rundläufertablettenpresse (Maschinensteuerung) sein. Es kann sich aber auch um eine von einer solchen Maschinensteuerung getrennte Steuereinrichtung handeln.

Weiterhin ist eine Antriebseinrichtung vorgesehen, die die Weiche abhängig von dem von der Steuereinrichtung erhaltenen Schaltsignal in die gewünschte Schaltstellung bewegt. Die Antriebseinrichtung kann zum Beispiel einen Elektromotor umfassen, der die Weiche zwischen der ersten und der mindestens einen zweiten Schaltstellung bewegt, zum Beispiel eine Weichenfahne verschwenkt. Denkbar wären aber zum Beispiel auch elektromagnetische Antriebe.

Erfindungsgemäß wird die Weiche beim Bewegen aus einer Ausgangsschaltstellung in eine Zielschaltstellung zunächst mit einer vorgegebenen Bewegungsgeschwindigkeit in Richtung der Zielschaltstellung bewegt. Beispielsweise wenn die Weiche bzw. eine Weichenfahne schwenkbar zwischen der Ausgangsschaltstellung und der Zielschaltstellung ist, kann die Bewegungsgeschwindigkeit entsprechend eine Drehgeschwindigkeit der Schwenkbewegung sein. Die vorgegebene Bewegungsgeschwindigkeit kann konstant sein oder auch ein Geschwindigkeitsprofil sein. Beispielsweise könnte die Bewegungsgeschwindigkeit der Weiche bzw. einer Weichenfahne ausgehend von der Ausgangsschaltstellung einmal oder mehrfach beschleunigt werden, beispielsweise kontinuierlich beschleunigt werden. Bevor die Weiche ihre Zielschaltstellung erreicht, wird die Bewegungsgeschwindigkeit der Weiche reduziert. Die Weiche wird anschließend weiter in ihre Zielschaltstellung bewegt.

Der Erfindung liegt der Gedanke zugrunde, dass ein Einklemmen von Tabletten zwischen der Weiche und beispielsweise einer gegenüberliegenden Wand des Tablettenablaufs verhindert werden kann, wenn die Weiche vor Erreichen ihrer Zielschaltstellung, und insbesondere vor Erreichen einer Stellung, in der eine Tablette von der Weiche eingeklemmt werden kann, in ihrer Bewegung verlangsamt wird. Aufgrund dieser Reduzierung der Bewegungsgeschwindigkeit wird sichergestellt, dass eine sich in einem für das Einklemmen durch die Weiche kritischen Bereich befindende Tablette ausreichend Zeit für das Abfließen hat bevor es zu einem Einklemmen durch die Weiche kommen kann. Wenn die Weiche nach dem Reduzieren der Bewegungsgeschwindigkeit anschließend weiter bis in ihre Zielschaltstellung bewegt wird, ist dieser von der Weiche überstrichene Bereich des Tablettenablaufs bereits frei von Tabletten. Ein Einklemmen von Tabletten beim Schalten der Weiche wird erfindungsgemäß also von vornherein vermieden. Indem erfindungsgemäß ein etwaiges Einklemmen von Tabletten vermieden wird, wird auch ein Tablettenstau in dem Tablettenablauf mit den erläuterten negativen Folgen sicher vermieden. Eine Weichenfehlfunktion, die in der Praxis eine der häufigsten Ursachen für den Stillstand einer Rundläufertablettenpresse ist, wird vermieden, so dass Folgeprobleme, wie ein Tablettenstau und die hiermit verbundenen Konsequenzen, ausbleiben.

Indem ein Tablettenstau vermieden wird, wird die Zuverlässigkeit und Verfügbarkeit der Rundläufertablettenpresse erhöht. Bedienpersonen werden durch das automatisierte Vermeiden der Weichenfehlfunktion vor toxischen Produkten und einer damit verbundenen Gesundheitsgefährdung geschützt. Manueller Aufwand zum Beheben einer Weichenfehlfunktion wird vermieden. Indem darüber hinaus ein Stillstand der Rundläufertablettenpresse und ein anschließender Anfahrvorgang vermieden werden können, wird entstehender Ausschuss im Vergleich zum Stand der Technik erheblich reduziert. Darüber hinaus kann eine erhöhte Tablettenqualität durch einen geringeren mechanischen Stress der Tabletten erreicht werden.

Die Weiche kann durch die Antriebseinrichtung, gesteuert durch die Steuereinrichtung, nach der Reduzierung der Bewegungsgeschwindigkeit beispielsweise mit konstant der reduzierten Bewegungsgeschwindigkeit weiter bis in die Zielschaltstellung bewegt werden. Um die Schaltzeit zu verringern, ist es aber auch möglich, dass die Bewegungsgeschwindigkeit der Weiche nach der Geschwindigkeitsverringerung durch die Antriebseinrichtung, gesteuert durch die Steuereinrichtung, wieder erhöht wird, die Weiche also wieder beschleunigt wird, beispielsweise bis auf eine vorgegebene Maximalgeschwindigkeit. Auch für die Bewegung der Weiche nach der Geschwindigkeitsverringerung bis zum Erreichen der Zielschaltstellung kann also mittels der durch die Steuereinrichtung gesteuerten Antriebseinrichtung ein Geschwindigkeitsprofil durchfahren werden. Beispielsweise könnte die Bewegungsgeschwindigkeit der Weiche bzw. einer Weichenfahne nach von der Geschwindigkeitsverringerung einmal oder mehrfach beschleunigt werden, beispielsweise kontinuierlich beschleunigt werden. In an sich bekannter Weise kann mindestens ein an sich bekannter Sensor vorgesehen sein, der mindestens eine, bevorzugt sämtliche Endstellungen der Weiche, also die erste und die mindestens eine zweite Schaltstellung bzw. die Ausgangsschaltstellung und die Zielschaltstellung detektiert. Das Detektieren der Endstellungen kann dabei direkt oder indirekt erfolgen. Beispielsweise kann der Sensor oder können die Sensoren auch aus anderen Messwerten auf das Erreichen bzw. Nichterreichen der jeweiligen Endstellung schließen. Als Sensoren kommen zum Beispiel Induktionssensoren oder Mikroschalter infrage, die das Erreichen bzw. Nichterreichen der jeweils vorgegebenen Endstellung erkennen. Es kann sich aber selbstverständlich auch um andere Sensoren handeln. Weiter beispielhaft genannt sei ein Winkelsensor, der die Winkellage der Weiche, beispielsweise einer Weichenfahne, erfasst und so alle Endstellungen und vorzugsweise auch beliebige Zwischenstellungen der Weiche erfassen kann. Ein solcher Winkelsensor ist besonders geeignet, wenn ein vorgegebenes Geschwindigkeitsprofil durchfahren werden soll. Auch kann mit einem solchen Winkelsensor in besonders einfacher Weise der Zeitpunkt für die Geschwindigkeitsverringerung der Weiche festgestellt werden. Bei einem Bewegen der Weiche aus einer Ausgangsschaltstellung, zum Beispiel der ersten Schaltstellung, in eine Zielschaltstellung, zum Beispiel der mindestens einen zweiten Schaltstellung, kann der mindestens eine Sensor detektieren, ob die Zielschaltstellung erreicht wurde oder nicht. Die Messsignale des mindestens einen Sensors können an der Steuereinrichtung anliegen.

Gemäß einer Ausgestaltung kann die Steuereinrichtung dazu ausgebildet sein, die Antriebseinrichtung derart anzusteuern, dass die Bewegungsgeschwindigkeit der Weiche vor Erreichen der Zielschaltstellung bis auf Null verringert wird und die Weiche anschließend weiter in die Zielschaltstellung bewegt wird. In diesem Extremfall erfolgt also eine Verringerung der Bewegungsgeschwindigkeit bis zum Stillstand. Die Verringerung der Bewegungsgeschwindigkeit erfolgt dabei derart, dass die Weiche, beispielsweise eine Weichenfahne, in ihrer Stillstandsposition eine sich gegebenenfalls zwischen der Weiche bzw. Weichenfahne und einer gegenüberliegenden Wand befindliche Tablette noch nicht einklemmen kann. In der Stillstandsposition der Weiche bzw. der Weichenfahne ist somit der Abstand zwischen der Weiche bzw. der Weichenfahne und beispielsweise einer gegenüberliegenden Wand größer als der Durchmesser der (größten) durch den Tablettenablauf geförderten Tablette. Beispielsweise kann dieser Abstand mindestens 10 mm, vorzugsweise mindestens 19 mm, weiter vorzugsweise mindestens 25 mm, betragen. Nach Erreichen des Stillstands wird die Weiche (weiter) in ihre Zielschaltstellung bewegt. Es besteht so ausreichend Zeit für etwaige in dem Zwischenbereich befindliche Tabletten zum Abfließen. Dabei kann eine definierte Stillstandszeit vorgegeben werden, nach deren Ablauf die Steuereinrichtung die Antriebseinrichtung zum weiteren Bewegen der Weiche in ihre Zielschaltstellung ansteuert. Es erfolgt dabei vorzugsweise keine Umkehrung der Bewegungsrichtung der Weiche.

Der Tablettenablauf kann wie an sich bekannt mindestens einen Ablaufkanal aufweisen. Insbesondere kann der Tablettenablauf mehrere Ablaufkanäle aufweisen, zwischen denen die Weiche den Tablettenstrom umlenken kann. Jeder Ablaufkanal kann zu einem der Tablettenausgänge führen. Wie bereits erläutert, können die Tablettenausgänge beispielsweise zu einem Ausgang für Schlechttabletten, für Guttabletten oder zu einer Messeinrichtung für einen Musterzug führen.

Die Weiche kann mindestens ein in dem mindestens einen Ablaufkanal des Tablettenablaufs schwenkbar zwischen der ersten Schaltstellung und der mindestens einen zweiten Schaltstellung gelagertes Weichenelement, vorzugsweise eine Weichenfahne, umfassen. Eine solche Weichenfahne ist ein länglicher Körper, der vorzugsweise im Bereich seines einen Endes schwenkbar an dem Ablaufkanal gelagert ist. Das gegenüberliegende freie Ende der Weichenfahne führt dann die maximale Schwenkbewegung aus. Das freie Ende der Weichenfahne kann in Ablaufrichtung der Tabletten gesehen vor der Schwenklagerung angeordnet sein. Die Weichenfahne kann zum Beispiel aus einem Metallwerkstoff bestehen. Dies ist an sich bekannt. Selbstverständlich kann die Weiche auch mehrere solcher, in Strömungsrichtung der Tabletten hintereinander angeordnete Weichenfahnen umfassen, um beispielsweise zwischen drei Ablaufkanälen schalten zu können.

Nach einer weiteren Ausgestaltung kann die Steuereinrichtung dazu ausgebildet sein, die Antriebseinrichtung derart anzusteuern, dass die Verringerung der Bewegungsgeschwindigkeit erfolgt, wenn zwischen dem mindestens einen Weichenelement, vorzugsweise der Weichenfahne, insbesondere dem freien Ende der Weichenfahne, und einer die Zielschaltstellung begrenzenden Innenwand des mindestens einen Ablaufkanals ein Abstand von mehr als 10 mm, vorzugsweise mehr als 19 mm, weiter vorzugsweise mehr als 25 mm, besteht. Auf diese Weise wird besonders zuverlässig sichergestellt, dass sich im Schaltbereich des Weichenelements befindende Tabletten noch rechtzeitig abfließen können.

Gemäß einer weiteren Ausgestaltung kann mindestens ein Ablaufkanal mindestens einen sich in Ablaufrichtung der Tabletten erweiternden, vorzugsweise sich stufenartig erweiternden, Abschnitt aufweisen. Der mindestens eine sich erweiternde Abschnitt kann sich gemäß einer weiteren Ausgestaltung in Ablaufrichtung der

Tabletten vor dem, vorzugsweise unmittelbar vor dem, von der Weichenfahne, beispielsweise ihrem freien Ende, bei ihrer Schwenkbewegung überstrichenen Bereich befinden. Weiterhin ist es vorteilhaft, wenn an gegenüberliegenden Wänden des Ablaufkanals jeweils mindestens ein sich erweiternder, vorzugsweise sich stufenartig erweiternder, Abschnitt vorgesehen ist. Die Erweiterung des mindestens einen sich erweiternden Abschnitts kann nach einer weiteren Ausgestaltung eine Breite von mindestens 10 mm, vorzugsweise mindestens 19 mm, weiter vorzugsweise mindestens 25 mm aufweisen. Die Breite wird in einer Richtung senkrecht zur Strömungsrichtung der Tabletten bzw. der Längsachse des Ablaufkanals gemessen. Die Breite ist dabei bevorzugt mindestens so breit wie die größte durch den Ablaufkanal zu fördernde Tablette.

Die vorgenannte vorzugsweise stufenartige Erweiterung bildet eine Schaltbucht. Insbesondere vergrößert sich hier die Breite des Ablaufkanals. Die Erweiterung kann sich im Bereich einer oder sämtlicher Zielschaltstellungen der Weiche befinden. Die Erweiterung ist derart ausgeführt, dass eine sich hierin befindliche Tablette den übrigen Tablettenstrom nicht behindert. Ein Tablettenstau wird besonders sicher verhindert.

Gemäß einer weiteren Ausgestaltung kann die Steuereinrichtung dazu ausgebildet sein, die Antriebseinrichtung derart anzusteuern, dass die Bewegungsgeschwindigkeit der Weiche unmittelbar vor Erreichen des sich erweiternden Abschnitts oder nach Erreichen des sich erweiternden Abschnitts verringert wird. Die Verlangsamung der Weiche kann also im Wesentlichen erst bei Erreichen des sich erweiternden Abschnitts erfolgen oder die Weiche kann sich zum Zeitpunkt des Eintritts der Verlangsamung bereits in dem sich erweiternden Abschnitt befinden. Sofern die Bewegungsgeschwindigkeit bis auf Null reduziert wird, kann der Stillstand wiederum unmittelbar vor Erreichen des sich erweiternden Abschnitts oder nach Erreichen des sich erweiternden Abschnitts eintreten, also wenn sich die Weiche, beispielsweise eine Weichenfahne, bereits innerhalb des sich erweiternden Abschnitts befindet. Durch die vorgenannten Maßnahmen wird verhindert, dass die Weiche, beispielsweise eine Weichenfahne, durch ihre Verlangsamung bzw. ihren Stillstand den Tablettenstrom beeinträchtigt. Vielmehr kann die Verlangsamung bzw. der Stillstand in oder an der erläuterten Schaltbucht erfolgen, so dass die übrigen Tabletten problemlos an der Weiche, beispielsweise einer Weichenfahne, vorbeiströmen können. Insbesondere kann vorgesehen sein, dass die Weiche, beispielsweise eine Weichenfahne, in ihrer Stillstandsposition im Wesentlichen nicht (nach innen) über den in Ablaufrichtung der Tabletten unmittelbar vor dem sich erweiternden Abschnitt befindlichen Führungsabschnitt des Ablaufkanals vorsteht. Dieser Führungsabschnitt kann durch eine Wand des Ablaufkanals oder bei mehreren hintereinander angeordneten Weichen bzw. Weichenfahnen auch durch eine in Ablaufrichtung vorgeordnete Weiche bzw. Weichenfahne gebildet sein.

Das erfindungsgemäße Verfahren wird mit einer erfindungsgemäßen Rundläufertablettenpresse durchgeführt. Entsprechend kann die erfindungsgemäße Rundläufertablettenpresse zur Durchführung des erfindungsgemäßen Verfahrens ausgebildet sein.

Ein Ausführungsbeispiel der Erfindung wird nachfolgend anhand von Figuren näher erläutert. Es zeigen schematisch:
- Figur 1: eine erfindungsgemäße Rundläuferpresse in einer abgewickelten Darstellung des Rotors,
- Figur 2: eine erfindungsgemäße Weiche der in Figur 1 gezeigten Rundläufertablettenpresse in einer ersten Betriebsstellung,
- Figur 3: die Weiche aus Figur 2 in einer zweiten Betriebsstellung,
- Figur 4: die Weiche aus Figur 2 in einer dritten Betriebsstellung, und
- Figur 5: die Weiche aus Figur 2 in einer vierten Betriebsstellung.

Soweit nichts anderes angegeben ist, bezeichnen in den Figuren gleiche Bezugszeichen gleiche Gegenstände.

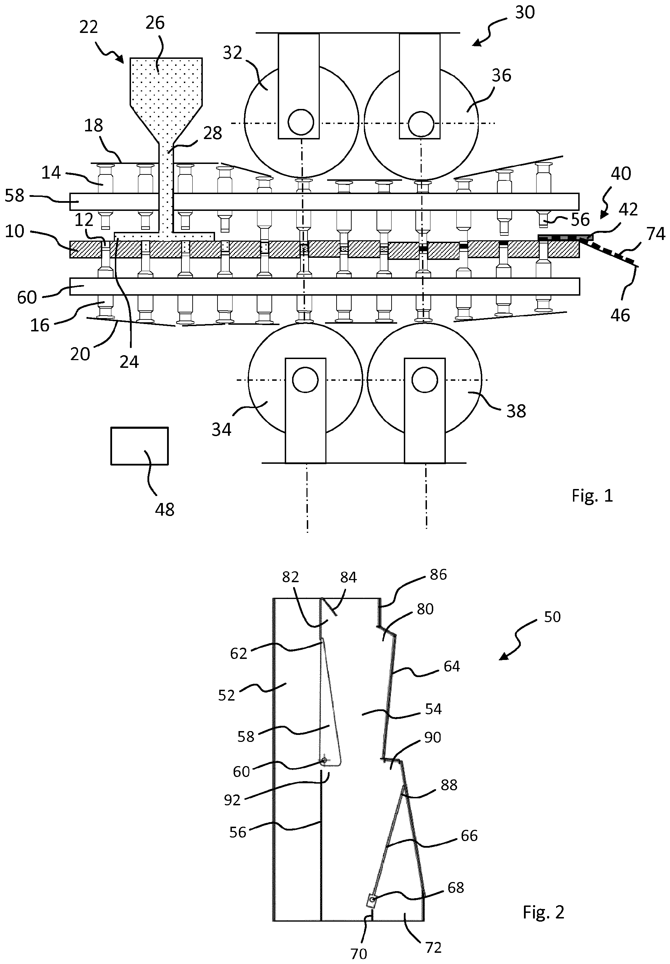

Die in Figur 1 gezeigte Rundläufertablettenpresse umfasst einen durch einen nicht näher dargestellten Drehantrieb drehend angetriebenen Rotor mit einer

Matrizenscheibe 10, die eine Mehrzahl von Kavitäten 12 aufweist. Die Kavitäten 12 können beispielsweise durch Bohrungen der Matrizenscheibe 10 gebildet sein. Weiter umfasst der Rotor eine Mehrzahl von oberen Stempeln 14 und unteren Stempeln 16, die mit der Matrizenscheibe 10 synchron umlaufen. Jeweils ein Paar aus oberem Stempel 14 und unterem Stempel 16 ist dabei einer Kavität 12 zugeordnet. Die axiale Bewegung der oberen Stempel 14 und unteren Stempel 16 im Zuge der Drehung des Rotors wird durch obere Steuerkurvenelemente 18 und untere Steuerkurvenelemente 20 gesteuert. Die Rundläufertablettenpresse umfasst weiterhin eine Fülleinrichtung 22, die eine Füllkammer 24 aufweist. Die Fülleinrichtung 22 umfasst darüber hinaus ein trichterförmiges Füllmaterialreservoir 26, das über einen Zuführabschnitt 28 mit der Füllkammer 24 in Verbindung steht. Auf diese Weise gelangt in dem vorliegenden Beispiel pulverförmiges Füllmaterial aus dem Füllmaterialreservoir 26 über den Zuführabschnitt 28 schwerkraftbedingt in die Füllkammer 24 und aus dieser über eine an der Unterseite der Füllkammer 24 vorgesehene Befüllöffnung wiederum schwerkraftbedingt in die Kavitäten 12 der Matrizenscheibe 10.

Außerdem umfasst die Rundläufertablettenpresse eine Druckeinrichtung 30. Die Druckeinrichtung 30 besitzt eine Vordruckeinrichtung mit einer oberen Vordruckrolle 32 und einer unteren Vordruckrolle 34 sowie eine Hauptdruckeinrichtung mit einer oberen Hauptdruckrolle 36 und einer unteren Hauptdruckrolle 38. Darüber hinaus umfasst die Rundläufertablettenpresse eine Auswurfeinrichtung 40, vorliegend mit einem Abstreifer 42, der die in der Rundläuferpresse hergestellten Tabletten 74 einem Tablettenablauf 46 zuführt.

Eine Steuereinrichtung zum Betrieb der Rundläuferpresse ist bei dem Bezugszeichen 48 gezeigt. Die Steuereinrichtung 48 ist über nicht näher dargestellte Leitungen unter anderem mit dem Drehantrieb des Rotors verbunden.

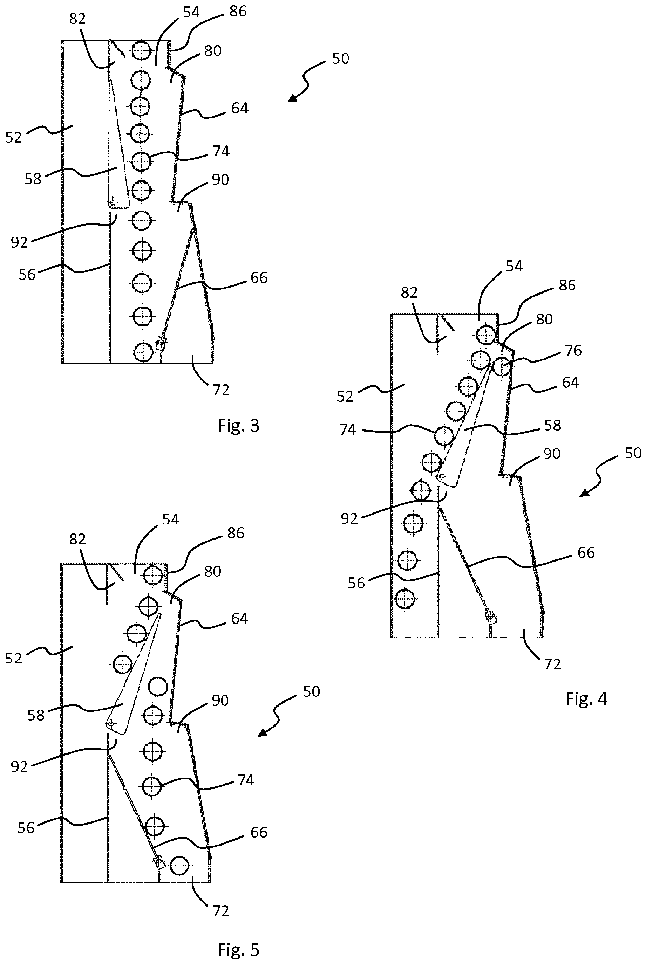

In dem Tablettenablauf 46 befindet sich eine in den Figuren 2 bis 5 dargestellte Weiche 50. Die Weiche 50 weist einen ersten Ablaufkanal 52 und einen zweiten Ablaufkanal 54 auf. Der erste und zweite Ablaufkanal 52, 54 sind durch eine Trennwand 56 voneinander getrennt. In der Trennwand 56 befindet sich eine erste Weichenfahne 58, die um eine erste Schwenkachse 60 schwenkbar zwischen der in Figur 2 gezeigten ersten Schaltstellung und einer nicht gezeigten zweiten Schaltstellung, in der das freie Ende 62 der Weichenfahne 58 an der gegenüberliegenden Wand 64 des zweiten Ablaufkanals 54 anliegt, gelagert ist. Tabletten fließen in den Figuren 2 bis 5 von oben nach unten durch die Weiche 50. Stromab der ersten Weichenfahne 58 ist in dem zweiten Ablaufkanal 54 eine zweite Weichenfahne 66 um eine zweite Schwenkachse 68 schwenkbar gelagert zwischen einer in Figur 2 gezeigten ersten Schaltstellung und einer in Figur 4 gezeigten zweiten Schaltstellung. Stromab der zweiten Weichenfahne 66 ist eine Trennwand 70 zu erkennen, die einen dritten Ablaufkanal 72 definiert. Zum Verschwenken der ersten Weichenfahne 58 und der zweiten Weichenfahne 66 zwischen ihrer jeweiligen ersten und zweiten Schaltstellung sind Antriebe einer Antriebseinrichtung vorgesehen, beispielsweise elektromotorische oder elektromagnetische Antriebe. Außerdem können nicht näher dargestellte, an sich bekannte Sensoren vorgesehen sein, die das Erreichen der jeweiligen Endstellungen der Weichenfahnen 58, 66, also der jeweiligen ersten Schaltstellung und der jeweiligen zweiten Schaltstellung, detektieren. Die Detektionssignale der Sensoren können in dem gezeigten Beispiel ebenfalls an der Steuereinrichtung 48 anliegen.

In den Figuren 3 bis 5 sind für unterschiedliche Betriebszustände der Weiche 50 durch die Weiche 50 ablaufende Tabletten 74 gezeigt. Wie in Figur 3 zu erkennen, laufen die Tabletten 74 bei in der ersten Schaltstellung befindlicher ersten Weichenfahne 58 und zweiten Weichenfahne 66 durch den zweiten Ablaufkanal 54 hindurch ab. Wird dagegen die erste Weichenfahne 58 in Richtung ihrer zweiten Schaltstellung bewegt, wie in Figur 4 gezeigt, wird der Tablettenstrom 74 aus dem zweiten Ablaufkanal 54 in den ersten Ablaufkanal 52 abgelenkt. Nur wenn sich die erste Weichenfahne 58 in ihrer ersten Schaltstellung befindet, gelangt der Tablettenstrom 74 zu der zweiten Weichenfahne 66. Diese lenkt den Tablettenstrom 74 dann je nach ihrer Schaltstellung in den dritten Ablaufkanal 72 ab (siehe Figur 5), oder verschließt den Zugang zu dem dritten Ablaufkanal 72, so dass der Tablettenstrom 74 weiter durch den zweiten Ablaufkanal 54 abfließen kann.

Der zweite Ablaufkanal 54 kann zum Beispiel zu einem Tablettenausgang für Guttabletten führen. Der dritte Ablaufkanal 72 kann zum Beispiel zu einem Tablettenausgang für einen Musterzug führen. Der erste Ablaufkanal 52 kann zum Beispiel zu einem Tablettenausgang für Schlechttabletten führen.

In Figur 4 ist beispielhaft eine zwischen der ersten Weichenfahne 58 und der gegenüberliegenden Wand 64 eingeklemmte Tablette 76 dargestellt. Aus diesem Grund kann die erste Weichenfahne 58 ihre zweite Schaltstellung nicht erreichen. Das Nichterreichen der zweiten Schaltstellung kann durch den jeweiligen Sensor detektiert werden und an die Steuereinrichtung ausgegeben werden. Diese gibt daraufhin eine Weichenfehlfunktion aus, die zu einem Stopp der Rundläufertablettenpresse führen kann.

Um dies zu vermeiden, ist erfindungsgemäß vorgesehen, dass die Bewegungsgeschwindigkeit der ersten Weichenfahne 58 und der zweiten Weichenfahne 66 bei einem Verstellen aus einer Ausgangsschaltstellung in eine Zielschaltstellung vor Erreichen der Zielschaltstellung verlangsamt wird, so dass eine sich etwaig in einem für ein Einklemmen kritischen Bereich befindliche Tablette 76 noch rechtzeitig abfließen kann bevor es zu einem Einklemmen kommt. Die jeweilige Weichenfahne 58, 66 kann entsprechend ihre Zielschaltstellung sicher erreichen und eine Weichenfehlfunktion wird vermieden.

Es kann beispielsweise vorgesehen sein, dass die erste Weichenfahne 58 und/oder die zweite Weichenfahne 66 vor Erreichen ihrer Zielschaltstellung bis zum Stillstand abgebremst wird. Beispielhaft ist dies in Figur 5 für die erste Weichenfahne 58 dargestellt. Die Weichenfahne 58 wurde ausgehend von ihrer ersten Schaltstellung in Richtung der zweiten Schaltstellung bewegt und vor Erreichen der zweiten Schaltstellung in die in Figur 5 dargestellte Stillstandsposition abgebremst. Nach Erreichen der Stillstandsposition und gegebenenfalls Ablauf eines vorgegebenen Stillstandszeitraums wird die Weichenfahne 58 weiter in Richtung ihrer zweiten Schaltstellung verstellt. Ein Einklemmen von Tabletten kann auf diese Weise besonders sicher vermieden werden.

In den Figuren 2 bis 5 ist außerdem zu erkennen, dass sich der zweite Ablaufkanal 54 unmittelbar vor dem von der ersten Weichenfahne 58 im Zuge ihrer Schwenkbewegung überstrichenen Bereich, insbesondere dem durch ihr freies Ende 62 überstrichenen Bereich, beidseitig in der Breite erweitert. Die sich erweiternden Abschnitte sind bei den Bezugszeichen 80 und 82 gezeigt. Dabei ist erkennbar, dass der sich erweiternde Abschnitt 82 dadurch gebildet ist, dass eine sich stromauf aufgrund eines Wandvorsprungs 84 befindende Verengung endet. Die sich im gezeigten Beispiel stufenartig erweiternden Abschnitte 80, 82 entsprechen in dem gezeigten Beispiel jeweils im Wesentlichen der Breite der durch die Weiche 50 abzuleitenden Tabletten 74, 76. Wie in Figur 5 beispielhaft für die erste Weichenfahne 58 dargestellt, ist diese unmittelbar bei Erreichen des sich erweiternden Abschnitts 80 zum Stillstand gebracht worden. Dadurch ist sichergestellt, dass die zum Stillstand gebrachte Weichenfahne 58 den weiteren Tablettenstrom 74 nicht beeinträchtigt. Insbesondere steht das freie Ende 62 der ersten Weichenfahne 58 im Wesentlichen nicht nach innen über den unmittelbar stromauf des erweiterten Abschnitts 80 befindlichen Wandabschnitt 86 hervor. Die diesbezüglich erläuterte Funktion ist im Übrigen für die erste Weichenfahne 58 und den gegenüberliegenden erweiterten Abschnitt 82 sowie für die zweite Weichenfahne 66 insoweit gleich. So befindet sich auch unmittelbar vor dem durch das freie Ende 88 der zweiten Weichenfahne 66 bei ihrer Schwenkbewegung überstrichenen Bereich in dem zweiten Ablaufkanal 54 auf gegenüberliegenden Seiten jeweils ein in der Breite erweiterter Abschnitt 90, 92. Der erweiterte Abschnitt 92 wird durch das Ende des eine Verengung des zweiten Ablaufkanals 54 bildenden, in Figur 2 unteren Endes der ersten Weichenfahne 58 gebildet. In gleicher Weise, wie oben zu der ersten Weichenfahne 58 erläutert, ermöglichen auch die erweiterten Abschnitte 90, 92 eine Verlangsamung bzw. einen Stillstand der zweiten Weichenfahne 66 beispielsweise bei Erreichen des jeweiligen erweiterten Abschnitts 90, 92, so dass der übrige Tablettenstrom 74 nicht beeinträchtigt wird. Wiederum entspricht dabei die Erweiterung 90, 92 im Wesentlichen in ihrer Breite dem Durchmesser der durch die Weiche 50 abzuleitenden Tabletten 74, 76.

### Bezugszeichenliste

- 10: Matrizenscheibe
- 12: Kavitäten
- 14: Obere Stempel
- 16: Untere Stempel
- 18: Obere Steuerkurvenelemente
- 20: Untere Steuerkurvenelemente
- 22: Fülleinrichtung
- 24: Füllkammer
- 26: Füllmaterialreservoir
- 28: Zuführabschnitt
- 30: Druckeinrichtung
- 32: Obere Vordruckrolle
- 34: Untere Vordruckrolle
- 36: Obere Hauptdruckrolle
- 38: Untere Hauptdruckrolle
- 40: Auswurfeinrichtung
- 42: Abstreifer
- 46: Tablettenablauf
- 48: Steuereinrichtung
- 50: Weiche
- 52: Erster Ablaufkanal
- 54: Zweiter Ablaufkanal
- 56: Trennwand
- 58: Erste Weichenfahne
- 60: Erste Schwenkachse
- 62: Freies Ende
- 64: Wand
- 66: Zweite Weichenfahne
- 68: Zweite Schwenkachse
- 70: Trennwand
- 72: Dritter Ablaufkanal
- 74: Tabletten
- 76: Tablette
- 80: Erweiterter Abschnitt
- 82: Erweiterter Abschnitt
- 84: Wandvorsprung
- 86: Wandabschnitt
- 88: Freies Ende
- 90: Erweiterter Abschnitt
- 92: Erweiterter Abschnitt

## Patentansprüche

1. Rundläufertablettenpresse, umfassend einen mittels eines Drehantriebs drehbaren Rotor, wobei der Rotor eine obere Stempelführung für obere Stempel (14) der Rundläufertablettenpresse und eine untere Stempelführung für untere Stempel (16) der Rundläufertablettenpresse sowie eine zwischen den Stempelführungen angeordnete Matrizenschreibe (10) aufweist, wobei die Stempel (14, 16) mit Kavitäten (12) der Matrizenscheibe (10) zusammenwirken, weiter umfassend eine Fülleinrichtung (22), durch die zu verpressendes Füllmaterial in die Kavitäten (12) der Matrizenscheibe (10) gefüllt wird, weiter umfassend mindestens eine obere Druckeinrichtung (30) und mindestens eine untere Druckeinrichtung (30), die im Betrieb mit den oberen Stempeln (14) und mit den unteren Stempeln (16) zum Verpressen des Füllmaterials in den Kavitäten (12) der Matrizenscheibe (10) zu Tabletten (74, 76) zusammenwirken, weiter umfassend eine Auswurfeinrichtung (40), in der in den Kavitäten (12) erzeugte Tabletten ausgeworfen werden, und umfassend einen Tablettenablauf (46), dem die ausgeworfenen Tabletten (74, 76) zugeführt werden, wobei in dem Tablettenablauf (46) mindestens eine Weiche (50) angeordnet ist, wobei eine Steuereinrichtung (48) vorgesehen ist, und wobei eine Antriebseinrichtung vorgesehen ist, die die Weiche (50) zwischen einer ersten Schaltstellung, in der Tabletten (74, 76) einem ersten Tablettenausgang zugeleitet werden und mindestens einer zweiten Schaltstellung, in der Tabletten (74, 76) mindestens einem zweiten Tablettenausgang zugeleitet werden, bewegt, **dadurch gekennzeichnet, dass** die Steuereinrichtung (48) dazu ausgebildet ist, die Antriebseinrichtung zum Bewegen der Weiche (50) aus einer Ausgangsschaltstellung in eine Zielschaltstellung derart anzusteuern, dass die Weiche (50) ausgehend von der Ausgangsschaltstellung mit einer Bewegungsgeschwindigkeit in Richtung der Zielschaltstellung bewegt wird, wobei die Bewegungsgeschwindigkeit der Weiche (50) vor Erreichen der Zielschaltstellung verringert wird.

2. Rundläufertablettenpresse nach Anspruch 1, **dadurch gekennzeichnet, dass** die Steuereinrichtung (48) dazu ausgebildet ist, die Antriebseinrichtung derart anzusteuern, dass ausgehend von der Ausgangsschaltstellung und/oder nach der Geschwindigkeitsverringerung bis zum Erreichen der Zielschaltstellung ein Geschwindigkeitsprofil durchfahren wird.

3. Rundläufertablettenpresse nach einem der vorhergehenden Ansprüche, **dadurch gekennzeichnet, dass** die Steuereinrichtung (48) dazu ausgebildet ist, die Antriebseinrichtung derart anzusteuern, dass die Bewegungsgeschwindigkeit der Weiche (50) ausgehend von der Ausgangsschaltstellung und/oder nach der Geschwindigkeitsverringerung einmal oder mehrfach beschleunigt wird.

4. Rundläufertablettenpresse nach einem der vorhergehenden Ansprüche, **dadurch gekennzeichnet, dass** die Steuereinrichtung (48) dazu ausgebildet ist, die Antriebseinrichtung derart anzusteuern, dass die Bewegungsgeschwindigkeit der Weiche (50) vor Erreichen der Zielschaltstellung auf Null verringert wird und die Weiche (50) anschließend weiter in die Zielschaltstellung bewegt wird.

5. Rundläufertablettenpresse nach einem der vorhergehenden Ansprüche, **dadurch gekennzeichnet, dass** der Tablettenablauf (46) mindestens einen Ablaufkanal (52, 54, 72) aufweist.

6. Rundläufertablettenpresse nach Anspruch 5, **dadurch gekennzeichnet, dass** die Weiche (50) mindestens ein in dem mindestens einen Ablaufkanal (52, 54, 72) des Tablettenablaufs (46) schwenkbar zwischen der ersten Schaltstellung und der mindestens einen zweiten Schaltstellung gelagertes Weichenelement, vorzugsweise eine Weichenfahne (58, 66), umfasst.

7. Rundläufertablettenpresse nach Anspruch 6, **dadurch gekennzeichnet, dass** die Steuereinrichtung (48) dazu ausgebildet ist, die Antriebseinrichtung derart anzusteuern, dass die Verringerung der Bewegungsgeschwindigkeit erfolgt, wenn zwischen dem mindestens einen Weichenelement und einer die Zielschaltstellung begrenzenden Innenwand des mindestens einen Ablaufkanals ein Abstand von mehr als 10 mm, vorzugsweise mehr als 19 mm, weiter vorzugsweise mehr als 25 mm, besteht.

8. Rundläufertablettenpresse nach einem der Ansprüche 5 bis 7, **dadurch gekennzeichnet, dass** mindestens ein Ablaufkanal (52, 54, 72) mindestens einen sich in Ablaufrichtung der Tabletten (74, 76) erweiternden, vorzugsweise sich stufenartig erweiternden, Abschnitt (80, 82, 90, 92) aufweist.

9. Rundläufertablettenpresse nach Anspruch 8, **dadurch gekennzeichnet, dass** sich der mindestens eine sich erweiternde Abschnitt (80, 82, 90, 92) in Ablaufrichtung der Tabletten (74, 76) vor dem, vorzugsweise unmittelbar vor dem, von dem Weichenelement bei seiner Schwenkbewegung überstrichenen Bereich befindet.

10. Rundläufertablettenpresse nach einem der Ansprüche 8 oder 9, **dadurch gekennzeichnet, dass** an gegenüberliegenden Wänden des Ablaufkanals (52, 54, 72) jeweils mindestens ein sich erweiternder, vorzugsweise sich stufenartig erweiternder, Abschnitt (80, 82, 90, 92) vorgesehen ist.

11. Rundläufertablettenpresse nach einem der Ansprüche 8 bis 10, **dadurch gekennzeichnet, dass** die Erweiterung des mindestens einen sich erweiternden Abschnitts (80, 82, 90, 92) eine Breite von mindestens 10 mm, vorzugsweise mindestens 19 mm, weiter vorzugsweise mindestens 25 mm aufweist.

12. Rundläufertablettenpresse nach einem der Ansprüche 8 bis 11, **dadurch gekennzeichnet, dass** die Steuereinrichtung (48) dazu ausgebildet ist, die Antriebseinrichtung derart anzusteuern, dass die Bewegungsgeschwindigkeit der Weiche (50) unmittelbar vor Erreichen des sich erweiternden Abschnitts (80, 82, 90, 92) oder nach Erreichen des sich erweiternden Abschnitts (80, 82, 90, 92) verringert wird.

13. Verfahren zum Betätigen einer Weiche (50) eines Tablettenablaufs (46) einer Rundläufertablettenpresse nach einem der vorhergehenden Ansprüche, bei dem die Weiche (50) zwischen einer ersten Schaltstellung, in der Tabletten (74, 76) einem ersten Tablettenausgang zugeleitet werden, und mindestens einer zweiten Schaltstellung, in der Tabletten (74, 76) mindestens einem zweiten Tablettenausgang zugeleitet werden, bewegt wird, **dadurch gekennzeichnet, dass** die Weiche (50) zum Bewegen aus einer Ausgangsschaltstellung in eine Zielschaltstellung ausgehend von der Ausgangsstellung mit einer Bewegungsgeschwindigkeit in Richtung der Zielschaltstellung bewegt wird, wobei die Bewegungsgeschwindigkeit der Weiche (50) vor Erreichen der Zielschaltstellung verringert wird.

14. Verfahren nach Anspruch 13, **dadurch gekennzeichnet, dass** ausgehend von der Ausgangsschaltstellung und/oder nach der Geschwindigkeitsverringerung bis zum Erreichen der Zielschaltstellung ein Geschwindigkeitsprofil durchfahren wird.

15. Verfahren nach einem der Ansprüche 13 oder 14 , **dadurch gekennzeichnet, dass** die Bewegungsgeschwindigkeit der Weiche (50) ausgehend von der .../5 Ausgangsschaltstellung und/oder nach der Geschwindigkeitsverringerung einmal oder mehrfach beschleunigt wird.

16. Verfahren nach einem der Ansprüche 13 bis 15, **dadurch gekennzeichnet, dass** die Bewegungsgeschwindigkeit der Weiche (50) vor Erreichen der Zielschaltstellung auf Null verringert wird und die Weiche (50) anschließend weiter in die Zielschaltstellung bewegt wird.

17. Verfahren nach einem der Ansprüche 13 bis 16, **dadurch gekennzeichnet, dass** es mit einer Rundläufertablettenpresse nach Anspruch 7 durchgeführt wird, wobei die Verringerung der Bewegungsgeschwindigkeit erfolgt, wenn zwischen dem mindestens einen Weichenelement und einer die Zielschaltstellung begrenzenden Innenwand des mindestens einen Ablaufkanals ein Abstand von mehr als 10 mm, vorzugsweise mehr als 19 mm, weiter vorzugsweise mehr als 25 mm, besteht.

18. Verfahren nach einem der Ansprüche 13 bis 17, **dadurch gekennzeichnet, dass** es mit einer Rundläufertablettenpresse nach einem der Ansprüche 9 bis 12 durchgeführt wird, wobei die Bewegungsgeschwindigkeit der Weiche (50) unmittelbar vor Erreichen des sich erweiternden Abschnitts (80, 82, 90, 92), vorzugsweise unmittelbar vor Erreichen des sich erweiternden Abschnitts (80, 82, 90, 92) oder nach Erreichen des sich erweiternden Abschnitts (80, 82, 90, 92) verringert wird.

## Claims

1. A rotary tablet press, comprising a rotor rotatable by means of a rotary drive, wherein the rotor has an upper punch guide for upper punches (14) of the rotary tablet press and a lower punch guide for lower punches (16) of the rotary tablet press as well as a die plate (10) arranged between the punch guides, wherein the punches (14, 16) interact with cavities (12) of the die plate (10), further comprising a filling apparatus (22), through which the filling material to be pressed is filled into the cavities (12) of the die plate (10), further comprising at least one upper pressing apparatus (30) and at least one lower pressing apparatus (30) which interact during operation with the upper punches (14) and with the lower punches (16) to press the filling material in the cavities (12) of the die plate (10) to form tablets (74, 76), further comprising an ejector apparatus (40), in which tablets generated in the cavities (12) are ejected, and comprising a tablet discharge (46) to which the ejected tablets (74, 76) are supplied, wherein at least one switch (50) is arranged in the tablet discharge (46), wherein a control apparatus (48) is provided, and wherein a drive apparatus is provided which moves the switch (50) between a first switching position, in which tablets (74, 76) are conducted to a first tablet outlet, and at least one second switching position, in which tablets (74, 76) are conducted to at least one second tablet outlet, **characterized in that** the control apparatus (48) is designed to control the drive apparatus to move the switch (50) from an initial switching position to a target switching position such that the switch (50) is moved, starting from the initial switching position, with a movement speed in the direction of the target switching position, wherein the movement speed of the switch (50) is reduced before the target switching position is reached.

2. The rotary tablet press according to claim 1, **characterized in that** the control apparatus (48) is designed to control the drive apparatus such that a speed profile is traveled through starting from the initial switching position and/or after the speed reduction until the target switching position is reached.

3. The rotary tablet press according to one of the preceding claims, **characterized in that** the control apparatus (48) is designed to control the drive apparatus such that the movement speed of the switch (50) is accelerated once or multiple times starting from the initial switching position and/or after the speed reduction.

4. The rotary tablet press according to one of the preceding claims, **characterized in that** the control apparatus (48) is designed to control the drive apparatus such that the movement speed of the switch (50) is reduced to zero before the target switching position is reached and the switch (50) is then moved further into the target switching position.

5. The rotary tablet press according to one of the preceding claims, **characterized in that** the tablet discharge (46) has at least one discharge channel (52, 54, 72).

6. The rotary tablet press according to claim 5, **characterized in that** the switch (50) comprises at least one switch element, preferably a switch flap (58, 66), mounted pivotably between the first switching position and the at least one second switching position in the at least one discharge channel (52, 54, 72) of the tablet discharge (46).

7. The rotary tablet press according to claim 6, **characterized in that** the control apparatus (48) is designed to control the drive apparatus such that the reduction in the movement speed takes place when there is a distance of more than 10 mm, preferably more than 19 mm, further preferably more than 25 mm, between the at least one switch element and an inner wall of the at least one discharge channel, which wall delimits the target switching position.

8. The rotary tablet press according to one of claims 5 to 7, **characterized in that** at least one discharge channel (52, 64, 72) has at least one portion (80, 82, 90, 92) expanding in the discharge direction of the tablets (74, 76), preferably expanding in steps.

9. The rotary tablet press according to claim 8, **characterized in that** the at least one expanding portion (80, 82, 90, 92) is located, in the discharge direction of the tablets (74, 76), before the, preferably immediately before the, region covered by the switching element during its pivoting movement.

10. The rotary tablet press according to one of claims 8 or 9, **characterized in that** at least one expanding portion (80, 82, 90, 92), preferably expanding in steps, is provided on each of the opposite walls of the discharge channel (52, 54, 72).

11. The rotary tablet press according to one of claims 8 to 10, **characterized in that** the expansion of the at least one expanding portion (80, 82, 90, 92) has a width of at least 10 mm, preferably at least 19 mm, further preferably at least 25 mm.

12. The rotary tablet press according to one of claims 8 to 11, **characterized in that** the control apparatus (48) is designed to control the drive apparatus such that the movement speed of the switch (50) is reduced immediately before the expanding portion (80, 82, 90, 92) is reached or after the expanding portion (80, 82, 90, 92) is reached.

13. A method for operating a switch (50) of a tablet discharge (46) of a rotary tablet press according to one of the preceding claims, in which the switch (50) is moved between a first switching position, in which tablets (74, 76) are conducted to a first tablet outlet, and at least one second switching position, in which tablets (74, 76) are conducted to at least one second tablet outlet, **characterized in that**, to move from an initial switching position to a target switching position, the switch (50) is moved, starting from the initial position, with a movement speed in the direction of the target position wherein the movement speed of the switch (50) is reduced before the target switching position is reached.

14. The method according to claim 13, **characterized in that** a speed profile is traveled through starting from the initial switching position and/or after the speed reduction until the target switching position is reached.

15. The method according to one of claims 13 or 14, **characterized in that** the movement speed of the switch (50) is accelerated once or multiple times starting from the initial switching position and/or after the speed reduction.

16. The method according to one of claims 13 to 15, **characterized in that** the movement speed of the switch (50) is reduced to zero before the target switching position is reached and the switch (50) is then moved further into the target switching position.

17. The method according to one of claims 13 to 16, **characterized in that** it is performed with a rotary tablet press according to claim 7, wherein the reduction in the movement speed takes place when there is a distance of more than 10 mm, preferably more than 19 mm, further preferably more than 25 mm, between the at least one switch element and an inner wall of the at least one discharge channel, which wall delimits the target switching position.

18. The method according to one of claims 13 to 17, **characterized in that** it is performed with a rotary tablet press according to one of claims 9 to 12, wherein the movement speed of the switch (50) is preferably reduced before the expanding portion (80, 82, 90, 92) is reached, preferably immediately before the expanding portion (80, 82, 90, 92) is reached, or after the expanding portion (80, 82, 90, 92) is reached.

## Revendications

1. Presse à comprimés rotative comportant un rotor susceptible d'être mis en rotation par un entraînement rotatif, dans laquelle le rotor présente un guide de poinçons supérieur pour des poinçons supérieurs (14) de la presse à comprimés rotative et un guide de poinçons inférieur pour des poinçons inférieurs (16) de la presse à comprimés rotative, ainsi qu'un disque à matrice (10) disposé entre les guides de poinçons, dans laquelle les poinçons (14, 16) coopèrent avec des cavités (12) dans le disque à matrice (10), comportant en outre un dispositif de remplissage (22) permettant de remplir un matériau de remplissage à comprimer dans les cavités (12) du disque à matrice (10), comportant en outre au moins un dispositif de pression supérieur (30) et au moins un dispositif de pression inférieur (30), lesquels coopèrent avec les poinçons supérieurs (14) et avec les poinçons inférieurs (16) pendant le fonctionnement pour presser le matériau de remplissage dans les cavités (12) du disque à matrice (10) de manière à former des comprimés (74, 76), comportant en outre un dispositif d'éjection (40) dans lequel des comprimés fabriqués dans les cavités (12) sont éjectés, et comportant une évacuation de comprimés (46) vers laquelle sont guidés les comprimés (74, 76) éjectés, dans laquelle au moins un commutateur (50) est disposé dans l'évacuation de comprimés (46), dans laquelle il est prévu un dispositif de commande (48), et dans laquelle il est prévu un dispositif d'entraînement chargé de déplacer le commutateur (50) entre une première position de commutation dans laquelle les comprimés (74, 76) sont guidés vers une première sortie de comprimés et au moins une deuxième position de commutation, dans laquelle des comprimés (74, 76) sont guidés vers au moins une deuxième sortie de comprimés, **caractérisée en ce que** le dispositif de commande (48) est conçu pour actionner le dispositif d'entraînement afin de déplacer le commutateur (50) d'une position de commutation initiale vers une position de commutation cible, de telle façon que le commutateur (50) est déplacé à partir de la position de commutation initiale à une vitesse de déplacement vers la position de commutation cible, la vitesse de déplacement du commutateur (50) étant réduite avant l'atteinte de la position de commutation cible.

2. Presse à comprimés rotative selon la revendication 1, **caractérisée en ce que** le dispositif de commande (48) est conçu pour actionner le dispositif d'entraînement de manière à parcourir un profil de vitesse à partir de la position de commutation initiale et/ou après la réduction de vitesse jusqu'à l'atteinte de la position de commutation cible.

3. Presse à comprimés rotative selon l'une des revendications précédentes, **caractérisée en ce que** le dispositif de commande (48) est conçu pour actionner le dispositif d'entraînement de telle façon que la vitesse de déplacement du commutateur (50) à partir de la position de commutation initiale et/ou après la réduction de vitesse est augmentée une fois ou à plusieurs reprises.

4. Presse à comprimés rotative selon l'une des revendications précédentes, **caractérisée en ce que** le dispositif de commande (48) est conçu pour actionner le dispositif d'entraînement de telle façon que la vitesse de déplacement du commutateur (50) avant l'atteinte de la position de commutation cible est réduite à zéro et le commutateur (50) est ensuite déplacé plus loin vers la position de commutation cible.

5. Presse à comprimés rotative selon l'une des revendications précédentes, **caractérisée en ce que** l'évacuation de comprimés (46) présente au moins un canal d'évacuation (52, 54, 72).

6. Presse à comprimés rotative selon la revendication 5, **caractérisée en ce que** le commutateur (50) comporte au moins un élément de commutateur, de préférence un drapeau de commutateur (58, 66), monté dans l'au moins un canal d'évacuation (52, 54, 72) de l'évacuation de comprimés (46) de façon pivotante entre la première position de commutation et l'au moins une deuxième position de commutation.

7. Presse à comprimés rotative selon la revendication 6, **caractérisée en ce que** le dispositif de commande (48) est conçu pour actionner le dispositif d'entraînement de telle façon que la réduction de la vitesse de déplacement s'effectue lorsqu'un espace supérieur à 10 mm, de préférence supérieur à 19 mm, de façon plus préférentielle encore supérieur à 25 mm, subsiste entre l'au moins un élément de commutateur et une paroi intérieure de l'au moins un canal d'évacuation délimitant la position de commutation cible.

8. Presse à comprimés rotative selon l'une des revendications 5 à 7, **caractérisée en ce qu'**au moins un canal d'évacuation (52, 54, 72) présente au moins une section (80, 82, 90, 92) s'élargissant dans la direction d'écoulement des comprimés (74, 76), s'élargissant de préférence par paliers.

9. Presse à comprimés rotative selon la revendication 8, **caractérisée en ce que** l'au moins une section (80, 82, 90, 92) s'élargissant se trouve en amont de la région balayée par l'élément de commutateur lors de son mouvement de pivotement, de préférence directement en amont de celle-ci, dans la direction d'écoulement des comprimés (74, 76).

10. Presse à comprimés rotative selon l'une des revendications 8 ou 9, **caractérisée en ce que** sur des parois opposées du canal d'évacuation (52, 54, 72), il est prévu respectivement au moins une section (80, 82, 90, 92) s'élargissant, de préférence par paliers.

11. Presse à comprimés rotative selon l'une des revendications 8 à 10, **caractérisée en ce que** l'élargissement de l'au moins une section (80, 82, 90, 92) s'élargissant présente une largeur d'au moins 10 mm, de préférence d'au moins 19 mm, de façon plus préférentielle d'au moins 25 mm.

12. Presse à comprimés rotative selon l'une des revendications 8 à 11, **caractérisée en ce que** le dispositif de commande (48) est conçu pour actionner le dispositif d'entraînement de telle façon que la vitesse de déplacement du commutateur (50) est réduite immédiatement avant l'atteinte de la section (80, 82, 90, 92) s'élargissant ou après l'atteinte de la section (80, 82, 90, 92) s'élargissant.

13. Procédé d'activation d'un commutateur (50) d'une évacuation de comprimés (46) d'une presse à comprimés rotative selon l'une des revendications précédentes, dans lequel le commutateur (50) est déplacé entre une première position de commutation dans laquelle les comprimés (74, 76) sont guidés vers une première sortie de comprimés, et au moins une deuxième position de commutation, dans laquelle des comprimés (74, 76) sont guidés vers au moins une deuxième sortie de comprimés, **caractérisé en ce que** pour le déplacement d'une position de commutation initiale vers une position de commutation cible, le commutateur (50) est déplacé à partir de la position de commutation initiale à une vitesse de déplacement vers la position de commutation cible, dans lequel la vitesse de déplacement du commutateur (50) est réduite avant l'atteinte de la position de commutation cible.

14. Procédé selon la revendication 13, **caractérisé en ce qu'**un profil de vitesse est parcouru à partir de la position de commutation initiale et/ou après la réduction de vitesse jusqu'à l'atteinte de la position de commutation cible.

15. Procédé selon les revendications 13 ou 14, **caractérisé en ce que** la vitesse de déplacement du commutateur (50) est augmentée une fois ou à plusieurs reprises à partir de la position de commutation initiale et/ou après la réduction de vitesse.

16. Procédé selon l'une des revendications 13 à 15, **caractérisé en ce qu'**avant l'atteinte de la position de commutation cible, la vitesse de déplacement du commutateur (50) est réduite à zéro et le commutateur (50) est ensuite déplacé plus loin vers la position de commutation cible.

17. Procédé selon l'une des revendications 13 à 16, **caractérisé en ce que** celui-ci est mis en œuvre à l'aide d'une presse à comprimés rotative selon la revendication 7, dans lequel la réduction de la vitesse de déplacement s'effectue lorsqu'un espace supérieur à 10 mm, de préférence supérieur à 19 mm, de façon plus préférentielle supérieur à 25 mm, subsiste entre l'au moins un élément de commutateur et une paroi intérieure de l'au moins un canal d'évacuation délimitant la position de commutation cible

18. Procédé selon l'une des revendications 13 à 17, **caractérisé en ce que** celui-ci est mis en œuvre à l'aide d'une presse à comprimés rotative selon l'une des revendications 9 à 12, dans lequel la vitesse de déplacement du commutateur (50) est réduite immédiatement avant l'atteinte de la section (80, 82, 90, 92) s'élargissant, de préférence immédiatement avant l'atteinte de la section (80, 82, 90, 92) s'élargissant ou après l'atteinte de la section (80, 82, 90, 92) s'élargissant.
